# EUROPÄISCHE PATENTSCHRIFT

(11) **EP 1 981 552 B1**
(45) Veröffentlichungstag und Bekanntmachung des Hinweises auf die Patenterteilung: **06.04.2011**
(21) Anmeldenummer: 07704469.1
(22) Anmeldetag: 09.02.2007
(51) Int. Cl.: A61L 24/02, A61L 24/04, A61L 27/44

(54) **RESORBIERBARES CALCIUMPHOSPHATBASIERENDES BIOPOLYMERVERNETZTES KNOCHENERSATZMATERIAL**
REABSORABABLE CALCIUM PHOSPHATE BASED BIOPOLYMER-CROSS-LINKED BONE REPLACEMENT MATERIAL
MATÉRIAU DE SUBSTITUTION OSSEUSE RÉSORBABLE À RÉTICULATION BIOPOLYMÈRE, À BASE DE PHOSPHATE DE CALCIUM

(30) Priorität: 09.02.2006 DE 102006006904
(43) Veröffentlichungstag der Anmeldung: 22.10.2008
(73) Patentinhaber: Dot Gmbh, D-18057 Rostock (DE)
(72) Erfinder: NEUMANN, Hans-Georg, 18146 Rostock (DE); KLINKENBERG, Ernst-Dieter, 18055 Rostock (DE); SCHAUER, Frieder, 17509 Lubmin (DE); LINDEQUIST, Ulrike, 17489 Greifswald (DE); JÜLICH, Wolf-Dieter, 17489 Greifswald (DE); MIKOLASCH, Annett, 17489 Griefswald (DE); MANDA, Katrin, 18581 Putbus (DE)
(74) Vertreter: Garrels, Sabine
(86) Internationale Anmeldenummer: PCT/EP2007/051251
(87) Internationale Veröffentlichungsnummer: WO 2007/090880

(56) Entgegenhaltungen:
- EP-A- 1 169 922
- WO-A-02/051449
- WO-A-2006/016809
- WO-A-2007/097710
- US-A- 5 015 677

## Beschreibung

### Technisches Gebiet

Die Erfindung betrifft ein resorbierbares Knochenersatzmaterial bestehend aus Calciumphosphat-Partikeln verschiedener Phasen, die in eine erfindungsspezifisch vernetzte Kollagenmatrix eingelagert sind.

### Stand der Technik

Knochendefekte, die nicht in natürlicher Heilung durch den Organismus geschlossen werden können, erfordern den Einsatz von Knochenersatzmaterialien, die natürlichen oder künstlichen Ursprungs sein können, den Defekt füllen und zu körpereigenem Knochen umbildet werden.

Den sogenannten "Goldenen Standard" stellt dabei die Nutzung von Eigenknochen dar, d.h. dem Patienten wird aus einem gesunden Bereich, z.B. aus dem Beckenkamm, ein Stück Knochen entnommen und zur Füllung des Defektes benutzt. Bei größeren Knochendefekten oder ungeeignetem Eigenknochen wird auch auf Fremdknochen zurückgegriffen, der entsprechend aufgearbeitet wird. Dem Vorteil, in größeren Mengen zur Verfügung zu stehen, steht bei Fremdknochen der Nachteil gegenüber, dass variierende Aufarbeitungsprozesse zu unterschiedlichen mechanischen Eigenschaften führen können (Palmer S.H., Gibbons C.L.M.H., Athanasou N.A.: JBJS (Br) 1999, 81-B, 333-5). Außerdem sind bei Fremdknochen Infektionen nicht auszuschließen (Boyce T., Edwards J., Scarborough N.: Orthop. Clin. North Am. 1999, 30(4), 571-81). Für die Nutzung von entbiologisiertem, tierischem Knochen treffen die gleichen Vorhalte wie für die Nutzung von Fremdknochen zu, so dass verschiedene synthetische Knochenersatzstoffe entwickelt wurden.

Die Vorteile der synthetischen Knochenersatzstoffe bestehen darin, dass neben der Verfügbarkeit die Kontrolle über die chemische Zusammensetzung und Struktur vorhanden ist und die Beeinflussung der biologisch wirksamen Eigenschaften derart vorgenommen werden kann, dass ein optimaler Therapieverlauf erreicht wird.

Die Mehrzahl der synthetischen Knochenersatzstoffe beinhaltet Calcium-Minerale wie, Calciumcarbonat, Calciumsulfat und verschiedene Calciumphosphate. In der Vergangenheit fanden insbesondere Calciumphosphate wie β-Tricalciumphosphat und Hydroxylapatit Anwendung, wobei Hydroxylapatit wesentlicher Bestandteil des natürlichen Knochens ist (Dorozhkin S.V., Epple M.: Angew. Chemie 2002, 114, 3260-77).

Knochenersatzmaterialien auf Calciumphosphatbasis lassen sich in verschiedenen Ausführungsformen für verschiedene Anwendungsbereiche herstellen. Granulate werden z.B. zur dentalen Knochendefektfüllung verwendet, während injizierbare Zemente bei der Stabilisierung von Wirbelkörpern Anwendung finden. Lasttragende Keramikformkörper werden bei Defekten des Schädels und der großen Röhrenknochen eingesetzt.

Im lasttragenden Fall ergeben sich für Knochenersatzmaterialien aus Calciumphosphaten besondere Schwierigkeiten. Die notwendige Struktur kann nur bestimmte Maximalkräfte aufnehmen und die natürlich vorhandene Sprödigkeit von synthetischen Calciumphospaten muss durch einen angepassten Herstellungsprozess kompensiert werden. Eine Erhöhung der Festigkeit wird z.B. durch Sinterung erreicht. Nachteilig ist dabei, dass gesinterte Calciumphospate wesentlich langsamer resorbiert werden als das dem normalen Therapieverlauf entsprechen würde (LeGeros R.Z.: Clinical Muterials 1993, 14, 65-88). Mit der Sinterung ist außerdem ein Verlust an Nanoporosität zu verzeichnen.

Eine weitere Möglichkeit, die Festigkeit von Calciumphosphat-basierten Knochenersatzmaterialien zu erhöhen, ist die Verdichtung durch kaltes isostatisches Pressen. Die Porosität dieser Formkörper geht dabei jedoch weitgehend verloren, so dass, um diesen Effekt zu kompensieren, eine makroskopische Strukturierung durch mechanische Nachbearbeitung, z.B. Bohrungen, erzeugt werden muss (D. Tadic D., M. Epple M.: Biomaterials 2003, 24, 4565-71).

Spielt die mechanische Belastbarkeit nicht die wesentliche Rolle, können Knochenersatzstoffe auf der Basis von Calciumphosphaten mit weiteren Verfahren hergestellt werden, die eine bessere Einstellung der Porosität gestatten. Beschränkt man sich auf anorganische Komponenten als Materialbestandteile, so sind insbesondere Sol-Gel-Prozesse gut geeignet, offenporige Netzwerkstrukturen zu erzeugen (Brinker C.J., Scherer G.W.: Sol-Gel Science: The Physics and Chemistry of Sol-Gel Processing, Academic Press, 1990).

Werden zusätzlich organische Komponenten als Binder benutzt, lassen sich Formkörper sowohl in direkt formabbildenden Prozessen erzeugen (Michnaa S., Wua W., Lewisa J.A. :Biomaterials 2005, 26, 5632-9), z.B. als räumliche Gitterstruktur als auch durch Pressformung (Weihe S., Wehmöller M., Tschakaloff A., von Oepen R., Schiller C., Epple M., Eufinger H.: Mund Kiefer Gesichts Chir 2001, 5 : 299 - 304) herstellen. Die Porosität dieser Formkörper muss durch den Formgebungsprozess selbst oder durch mechanische Bearbeitung eingestellt werden (D. Tadic D., M. Epple M.: Biomaterials 2003, 24, 4565-71).

Durch die Nutzung von Knochenersatzstoffen, die einstellbare Anteile von Caliciumphosphaten mit unterschiedlichen Löslichkeiten in der Körperflüssigkeit und damit verschiedenen Resorptionsraten beinhalten, kann der Resorptionsverlauf definiert gestaltet werden. Dadurch wird die Belastung über einen Zeitraum, in dem sich neues Knochengewebe bildet und festigt, vom Knochenersatzmaterial mit getragen. Die Calciumphosphate mit geringer Löslichkeit werden erst später, während der Remodellierung des Knochens, vollständig resorbiert (LeGeros R.Z.: Clinical Muterials 1993, 14, 65-88).

Zusammenfassend ist festzustellen, dass calciumphosphatbasierte Knochenersatzmaterialien zur Behandlung von einfachen Defekten erfolgreich benutzt werden. Ein Einsatz bei schweren Defekten erfolgt aber, bedingt durch materialtechnische Probleme, derzeit nicht. Diese Probleme liegen vor allem darin, das die aktuellen Verfahren nicht in der Lage sind, wesentliche Anforderungen an das Knochenersatzmaterial bzw. an aus ihm geformte defektfüllende Körper zu erfüllen;

(1) zum einen formschlüssige, d. h. anatomisch bzw. dem Defekt entsprechend geformte Knochenersatz-Formkörper herzustellen, die den Knochendefekt perfekt und auch kraftschlüssig ausfüllen, und

(2) andererseits eine leicht resorbierbare Struktur zu realisieren.

Außerdem ist es bei Nutzung der aktuellen Herstellungsverfahren sehr schwierig, zusätzliche organische oder anorganische Stoffe mit stark variierenden Konzentrationen in das Knochenersatzmaterial einzufügen sowie eine Verbesserung der lasttragenden Eigenschaften durch eingebettete permanente bzw. resorbierbare Stützstrukturen vorzunehmen.

WO 02/051449 A beschreibt ein Knochenersatzmaterial, das aus einem Substrat, einem Knochenwachstumsprotein, einer Calcium- und einer Phosphatquelle besteht. Als wesentlicher Bestandteil wird das Knochenwachstumsprotein angesehen, das aus Proteinen oder Proteinmischungen besteht, die entweder aus natürlichen Knochen oder durch rekombinante DNA-Techniken gewonnen werden und das Knochenwachstums gezielt anregen.

WO 2007/097710 A1 offenbart ein Knochenzement bestehend aus einem phenolgruppeenthaltenden, härtbaren Bindemittel das Trihydroxyaromaten umfasst, ein Füller wie Calciumphosphat und ein zusätzlicher Bestandteil wie Kollagen. Zum Aushärten der Zementkomposition kann ein Enzym eingesetzt werden.

Es sind jedoch keine befriedigenden Lösungen bekannt, die es ermöglichen Knochenersatzstoffe mit frei wählbaren DihydroxyVerbindungen als Brückenmolekül mit Collagen und Calciumphosphat-Partikeln verschiedener Phasen zu einer festen Matrix zu verbinden.

### Darstellung der Erfindung

Die erfindungsgemäße Aufgabestellung besteht darin, einen formschlüssigen, d.h. einen anatomisch bzw. dem Defekt entsprechend geformten nicht spröden Knochenersatz-Formkörper herzustellen, der den Knochendefekt perfekt ausfüllt und zudem resorbierbar ist.

Die Lösung der Aufgabenstellung wird durch die Herstellung des Knochenersatzstoffes aus einer Mischung von Calciumphoshatpartikeln eingebettet in eine erfindungsgemäß vernetzte Kollagenmatrix erreicht.

Insbesondere wird die Kollagenvernetzung durch eine Laccase-induzierte Peptidvernetzung und geeignete Brückenmoleküle erreicht.

Als Brückenmoleküle eignen sich grundsätzlich substituierte Dihydroxyaromaten und/oder Substrate der lignolytischen Polyphenoloxidasen, wie Laccasen.

Deshalb werden monocyclische ortho-Dihydroxyaromaten, monocyclische para-Dihydroxyaromaten, bicyclische Monohydroxyaromaten, polycyclische Monohydroxyaromaten, bicyclische Dihydroxyaromaten, polycyclische Dihydroxyaromaten, bicyclische Trihydroxyaromaten, polycyclische Trihydroxyaromaten, oder deren Mischungen eingesetzt. Die erfindungsgemäßen Hydroxyaromaten sind im Gegensatz zu den bekannten Muschelklebstoffen nicht Bestandteil einer Polymerkette.

Diese Aromaten können weiter substituiert sein. Bevorzugte funktionelle Gruppen sind Substituenten ausgewählt aus der Gruppe bestehend aus Halogen, Sulfo-, Sulfon-, Sulfamindo, Sulfanyl-, Amino-, Amido-, Azo-, Immino- und Hydroxy. Dabei ist festzustellen, dass substituierte Aromaten, insbesondere substituierte Dihydroxyaromaten, sehr günstige Polymerisationsegenschaften wie eine schnelle Polymerisation, eine geringere Eigenkopplung und eine gute Festigkeit der Vernetzung aufweisen. Eine geeignete Substitution der Aromaten führt dazu , dass auch Monohydroxyaromaten als Brückenmolekül zur Vernetzung geeignet sind. Substitution bedeutet im Rahmen dieser Erfindung, dass an den Aromaten neben den Hydroxylgruppen noch 1, 2, 3 oder 4 weitere Reste gebunden sind. Weiterhin sind auch monohydroxylierte Biarylverbindungen als Brückenmoleküle geeignet.

Besonders bevorzugt als Brückenmolekül sind Phenolderivate, die eine Hydroxygruppe oder eine Methoxygruppe an der ortho- oder para-Position aufweisen, die den Formeln 1 und 2 entsprechend

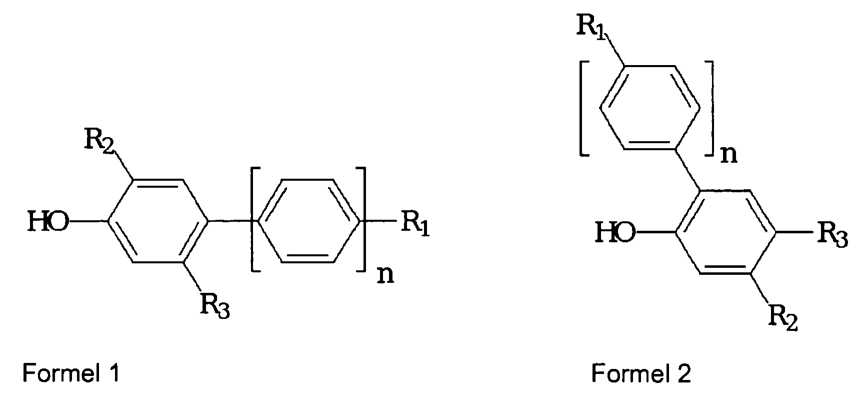

wobei
n = 0 - 10, bevorzugt 0 oder 1, insbesondere 0,
R₁ = OH oder NH₂ oder Hal, bevorzugt OH, Cl oder Br, insbesondere OH,
R₂ = H, CH₃, CHO, COCH₃, CONH₂, CON-Alkyl, CON-Alkyl-OH, COOH, COO-Alkyl, Alkyl, substituierter Aromat, insbesondere CON-Alkyl oder COO-Alkyl, und
R₃ = H, CH₃, Alkyl, substituierter Aromat, insbesondere H oder CH₃,
ist.

Alkyl bedeutet hier verzweigte oder unverzweigte aliphatische Kohlenwasserstoffketten, bevorzugt mit 1-20 Kohlenstoffatomen, mehr bevorzugt mit 1-6 Kohlenstoffen, z.B. Methyl, Ethyl, Propyl, Butyl, Isobutyl, n-Pentyl, n-Hexyl.

Mögliche Brückenmolküle sind weiterhin Verbindungen der Formel 1 und davon das Hydrochinon, welches weiter substituiert sein kann. Unter dem Aspekt einer möglichst schnellen Klebereaktion sind substituierte Dihydroxyaromaten mit geringer Eigenkopplung erfindungsgemäß besonders geeignet. Bevorzugt werden 2,5-Dihydroxybenzamide eingesetzt, besonders bevorzugt wird 2,5-Dihydroxy-N-(2-hydroxyethyl)-benzamid.

Im Fall der Trihydroxyaromaten ist es bevorzugt, dass nicht mehr als zwei Hydoxygruppen pro Benzoleinheit vorliegen. Besonders bevorzugt sind Polyphenyle, d.h. Biphenyl oder Triphenyl, folgender Formel 3:

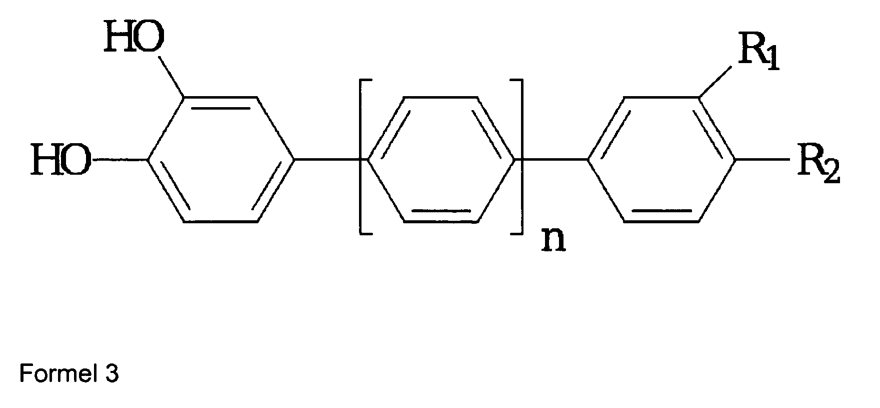

wobei
n = 0 - 10, bevorzugt 0 oder 1
und
R₁ = H und R₂ = OH oder R₁ = OH und R₂ = H
ist.

Dabei können die Phenyle der Formel 3 substituiert sein, z.B. in ortho-Position zu einer OH-Gruppe mit CH₃, CHO, COCH₃, CONH₂, CON-Alkyl, CON-Alkyl-OH, COOH, COO-Alkyl, Alkyl, substituierter Aromat, insbesondere CON-Alkyl oder COO-Alkyl, und / oder in metha-Position zu einer OH-Gruppe mit CH3, Alkyl, substituierter Aromat, insbesondere CH₃.

Die Vernetzung von Kollagen erfolgt erfindungsgemäß unter dem Einfluss von Polyphenoloxidasen, wie lignolytische Polyphenoloxidasen, insbesondere Laccasen (EC 1.10.3.2). Laccasen sind als Vernetzer bekannt. Sie können aus Pflanzen, Pilzen, Bakterien oder Insekten stammen oder von natürlichen Enzymen abgeleitet sein. Die im Rahmen dieser Erfindung zu verwendenden Laccasen können rekombinant hergestellt oder aufgereinigt sein. Beispiele sind Laccasen gewonnen aus den Gattungen Aspergillus, Neurospora, Podospora, Botrytis, Collybia, Fomes, Lentinus, Pleurotus, Pycnoporus, Pyricularia, Trametes, Rhizoctonia, Coprinus, Psatyrella, Myceliophthora, Schtalidium, Polyporus, Phlebia oder Coriolus. Die Herstellung von Laccasen ist in EP0947142 offenbart.

Durch den Einsatz von Polyphenoloxidasen, wie lignolytischen Polyphenoloxidasen, vorzugsweise Laccase (EC 1.10.3.2) kann deren Substratspektrum für die Vernetzungsreaktion ausgenutzt werden. Daher zeichnet sich die Erfindung insbesondere dadurch aus, dass für die Vernetzung ein weites Spektrum von Brückenmolekülen eingesetzt werden kann.

Variationen der Konzentrationen sind sowohl bei der Einzelkomponente Kollagen als auch beim Brückenmolekülstoff etwa von 1-50 mM möglich.

Dabei muss beachtet werden, dass je nach dem ausgewählten Brückenmolekül auch eine störende Eigenreaktion des Brückenmoleküls stattfindet, die die Bildung von vernetzenden Bindungen mindert. Eine zu geringe Konzentration der Brückenmoleküle führt zu einer zu langsamen Reaktion, eine zu hohe Konzentration führt zu stärkeren Nebenreaktionen durch Eigenkopplung. Die Konzentration der Polyphenoloxidase beeinflusst die Geschwindigkeit der Reaktion, wobei, je nach Anwendung durch Variation der Konzentration eine schnellere Vernetzung oder eine längere Verarbeitbarkeit der Kombination erreicht werden kann. Eine bevorzugtes Mengenverhältnis für des Vernetzen von Weichteilgeweben ist beispielsweise im Ansatz Kollagen 8,5 mM; 2,5-Dihydroxy-N-(2-hydroxyethyl)-benzamid 12,5 mM; Polyphenoloxidase: 0,32 U (156 nmol ml⁻¹ min⁻¹) realisiert.

Die Vernetzung einer Kollagenmatrix durch Brückenmoleküle, bei Einbettung von Calciumphosphat-Partikeln verschiedener Phasen und Konzentrationen in die Matrix, führt zu einem festen Material mit Eignung als Knochenersatzstoff.

Die Polyphenoloxidase und die Polyphenole werden bevorzugt in Phosphatpuffer wie Calciumphosphat oder Natriumphosphatpuffer oder PBS gelöst. Die Konsistenz der eingesetzten Komponenten Laccase und Polyphenole ist flüssig bis pastös. Die Viskosität der verwendeten Einzelkomponenten kann durch Lösungsmittel variiert werden. Die Konzentration der Lösungsmittel beeinflusst die Vernetzung.

Die Vernetzungsreaktion erfolgt bei bevorzugt pH-Wert 5-7. Die Reaktion kann im Temperaturbereich von 2-80°C ablaufen, bevorzugt ist jedoch eine Temperatur im Bereich von 20-37°C, insbesondere im Bereich von 25-30°C.

Zur Unterstützung der Vernetzung können weitere kürzerkettige Peptide eingesetzt werden. In einer besonders bevorzugten Ausführungsform der Erfindung sind etwa 50% der Aminosäuren des Peptids Lysin. Lysin und eine weitere Aminosäure können als sich wiederholende Dipeptid-Einheit angeordnet sein. Auch eine andere Abfolge oder die Aufnahme weiterer Aminosäuren, insbesondere von Arginin, Asparagin, Glutamin oder Histidin (anstelle von Lysin oder zusätzlich), Serin oder Threonin (anstelle von Tyrosin oder zusätzlich), von Cystein oder anderen Aminosäuren ist möglich.

In einer ebenfalls bevorzugten Ausführungsform handelt es sich um ausschließlich aus zwei Aminosäuren bestehende Polymere, beispielsweise (Lysin-Thyrosin)_{n,} wobei n = Werte zwischen 5 und 40, wie 5, 10 oder 20, annehmen kann.

Vorteilhaft wird weiterhin (Lysin-Thyrosin)ₙ als Peptit eingesetzt. Bei Verwendung von (Lysin-Thyrosin)ₙ in der Matrix ist Lysin ist im Knochenersatzmaterial in hoher Konzentration vorhanden und beeinflusst die Knochenzellproliferation und -differenzierung positiv.

Vor der Vernetzung besitzt das Stoffgemisch viskose Eigenschaften und lässt sich beliebig ausformen. Dabei sind sowohl einfache Formen wie Kügelchen als auch komplexe Formkörper darstellbar.

Weiterhin ist es möglich poröse Formkörper mit dem viskosen Material zu füllen und zu verfestigen.

In einer bevorzugten Ausführungsform wird das Material als interkonnektierend poröser Körper ausgeführt.

Bevorzugt beträgt der Gewichtsanteil am Gesamtgewicht von Calciumphosphat-Partikeln 70 bis 95 %, von Kollagen und gegebenenfalls Oligopeptiden 5-15 %, und von der Brückenmolekülsubstanz etwa 0,5 bis 5%.

Die erfindungsgemäßen Knochenersatzmaterialien können weitere Stoffe enthalten. In einer bevorzugten Ausführungsart enthält das Material zusätzlich SiO₂.

Das Knochenersatzmaterial kann mit Antibiotika dotiert werden. Dabei werden Antibiotika mit freien Aminogruppen, die dem Knochenersatzstoff vor der Vernetzung zugemischt wurden, während der Laccase-induzierten Vernetzung an Kollagen gebunden. Dabei konnte gezeigt werden, dass die auf diese Art mit Antibiotika dotierten Kollagene eine starke antimikrobielle Wirksamkeit aufweisen.

Das Knochenersatzmaterial kann mit und ohne Stützstruktur eingesetzt werden. Wird als Stützstruktur ein resorbierbares organisches Material eingesetzt, ist es erfindungsgemäß von besonderem Vorteil ein freie-Aminogruppen-tragendes organisches Material auszuwählen. In diesem Fall erfolgt die Vernetzung durch Brückenmoleküle nicht nur zum Kollagen sondern auch zur Stützstruktur, so das eine feste Verbindung erhalten wird, die aber im Laufe des Heilungsprozesses durch körpereigenes Gewebe ersetzt wird. Der Mechanismus der Resorption kann hydrolytisch oder enzymatisch sein. Insbesondere können die Peptide gespalten und einzelne Fragmente abtransportiert und ausgeschieden werden. Alternativ können Fragmente oder Aminosäuren auch in das sich regenerierende Gewebe eingebaut werden.

In einer weiteren Ausführungsform ist die Porosität des Calciumphosphat-Formkörpers gerichtet. Deshalb sind die mechanischen Eigenschaften des resultierenden Verbundformkörpers ebenfalls richtungsabhängig..

Durch Vernetzung der kontaktierenden Oberflächen zweier Formkörper mit richtungsabhängigen mechanischen Eigenschaften, die zu einer festen Verbindung der Formkörper führt, wird die Richtungsabhängigkeit der mechanischen Eigenschaften des resultierenden Formkörpers reduziert.

Durch die feste Verbindung mehrerer flächiger Formkörper durch die Vernetzung ihrer paarweise kontaktierenden Oberflächen entsteht ein mehrlagiger Verbundformkörper, der eine hohe mechanischen Belastbarkeit aufweist.

### Weg(e) zur Ausführung der Erfindung

1. Beispiel

Eine Mischung aus Partikeln verschiedener Calciumphosphatmodifikationen und Kollagen wird homogen mit 2,5-Dihydroxy-N-(2-hydroxyethyl)-benzamid vemischt, so dass eine Konzentration von 12,5 mM erreicht wird. Die Vernetzung wird durch Zugabe von Laccase mit einer Aktivität von 0,32 U (156 nmol ml⁻¹ min⁻¹) ausgelöst. Nach der Aushärtung des Klebers bilden die mineralischen Bestandteile zusammen mit dem Klebermaterial in Abhängigkeit von den Feststoffanteilen ein hochviskoses oder festes Material.

2. Beispiel

Es wird eine Versuchsanordnung wie im Beispiel 1 gewählt, jedoch wird zusätzlich zum Kollagen ein Oligopeptid (2 bis ca. 100 Aminosäuren Länge, bevorzugt ca. 4 bis ca. 20 Aminosäuren Länge) hinzugefügt. Neben den üblichen proteinogenen Aminosäuren können auch modifizierte bzw. untypische Aminosäuren wie Hydoxylysin in dem Oligopeptid enthalten sein. Bevorzugt werden Lysin-haltige Oligopeptide eingesetzt, besonders vorteilhaft ist es, wenn etwa 50% der Aminosäuren des Peptids Lysin sind. Zusätzlich können etwa 50% der Aminosäuren des Peptids Tyrosin sein. Lysin und Tyrosin können z.B. als sich wiederholende Dipeptid-Einheit angeordnet sein. Insbesondere mit einem Zusatz von Peptiden, die aus sich wiederholenden Dipeptideinheiten von Lysin und Tyrosin bestanden ([Lys-Tyr]ₙ oder [Tyr-Lys]ₙ, n=5 oder n=10), wurde eine gute Vernetzung unter Einbeziehung des Kollagens und unter Einschluss der Calciumphosphate erreicht.

3. Beispiel

Die in Beispiel 2 beschriebenen Oligopeptide werden in PBS (Phosphate Buffered Saline, 2,7 M NaCl, 54 mM KCI, 87 mM Na₂HPO₄ 30 mM KH₂PO₄ pH 7.4) aufgenommen und Laccase wird zugegeben (Komponente 1). 2,5-Dihydroxy-N-(2-hydroxyethyl)-benzamid wird in PBS gelöst (Komponente 2). Nach Vereinigung der beiden Komponenten erfolgt die Vermischung mit Partikeln verschiedener Calciumphosphatmodifikationen und Kollagen. Die Menge an Lösungsmittel wird minimal gewählt, um eine möglichst konzentrierte Lösung der Komponenten zu erreichen.

4. Beispiel

In einen ungesinterten interkonnektierend porösen spröden Calciumphosphat-basierenden Formkörper wird die viskose Mischung aus Partikeln verschiedener Calciumphosphatmodifikationen und Kollagen, gegebenenfalls weiteren Oligopeptiden gemäß Beispiel 2, 2,5-Dihydroxy-N-(2-hydroxyethyl)-benzamid und Laccase durch Kapillarwirkung, durch Druckbeauflagung oder Unterdruck in die Poren eingebracht und bildet nach Aushärtung im Verbund mit dem CaP-Formkörper einen festen Verbundformkörper. In einer speziellen Ausführungsform ist der interkonnektierend poröse Körper derart ausbildet, dass dessen innere Oberfläche in hohem Maße durch Kollagen bedeckt ist, das örtlich bestimmt, vernetzt ist.

Nach der durch Laccase initiierten Vernetzung wird ein interkonnektierend poröses Material sowohl hinsichtlich des Minerals als auch des Kollagens erhalten. Implantiert fungiert dieses Material durch seine osteokonduktive Wirkung als biologische Leitschiene zum Aufbau fehlendes Knochens und damit zur Heilung des Defektes.

5. Beispiel

Es wird eines Verbundformkörpers nach Ausführungsbeispiel 3 hergestellt mit der Besonderheit, dass durch eine mehrlagige Vernetzung kontaktierender Oberflächen ein einfach oder mehrfach offener oder ein geschlossener Hohlkörper entsteht. Nach Implantation erfolgt die Regeneration des Knochendefekts bei Beibehaltung der Formstabilität.

6. Beispiel

Herstellung eines Knochenersatzstoffes nach Beispiel 1 mit der Besonderheit, dass in die Mischung vor Zugabe der Laccase ein Aminogruppen-tragende Antibiotikum homogen verteilt wurde. Nach der Vernetzung mit der Laccase ist das Kollagen mit dem Antibiotikum dotiert. Es entsteht also ein Knochenersatzstoff, der sowohl im Inneren als auch an der Oberfläche wirkstoffbeladen ist.

7. Beispiel

Herstellung eines mehrlagigen Verbundformkörpers nach Beispiel 6 mit der Besonderheit, das in eine oder mehrere Lagengrenzen gleiche oder verschiedene Wirkstoffe eingelagert werden.

## Patentansprüche

1. Resorbierbares Knochenersatzmaterial bestehend aus Calciumphosphat-Partikeln verschiedener Phasen und Kollagen **dadurch gekennzeichnet, dass**
a) das Kollagen über Brückenmoleküle, ausgewählt aus einer Gruppe, die einen monozyklischen Dihydroxyaromaten, einen bizyklischen Dihydroxyaromaten, einen polyzyklischen Dihydroxyaromaten, einen bizyklischen Trihydroxyaromaten oder einen polyzyklischen Trihydroxyaromaten umfasst, wobei die Hydroxyaromaten nicht Bestandteil einer Polymerkette sind, vernetzt ist, so dass eine Matrix ausgebildet wird, in die zusätzlich kürzerkettige Peptide, insbesondere Lysin enthaltende Peptide eingebettet sind, und
b) die Ausbildung der Kollagenmatrix mit darin eingebetteten Calciumphosphat-Partikeln verschiedener Phasen und Konzentrationen unter dem Einfluss von Polyphenoloxidasen erfolgt.

2. Resorbierbares Knochenersatzmaterial nach Anspruch 1, **dadurch gekennzeichnet, dass**
die Lysin enthaltenden Peptide sich wiederholende Lysin enthaltende Dipeptid-Einheiten sind.

3. Resorbierbares Knochenersatzmaterial nach Anspruch 1 oder 2, **dadurch gekennzeichnet, dass**
als kürzerkettige Peptide ausschließlich aus zwei Aminosäuren bestehende Polymere, in besonderer Weise vorteilhaft (Lysin-Thyrosin)ₙ, eingebaut werden, wobei n = Werte zwischen 5 und 40 annimmt.

4. Resorbierbares Knochenersatzmaterial nach Anspruch 1 bis 3 **dadurch gekennzeichnet, dass**
das Knochenersatzmaterial einen interkonnektierend porösen Körper bildet.

5. Resorbierbares Knochenersatzmaterial nach Anspruch 4 **dadurch gekennzeichnet, dass**
es als Formkörper ausgeführt ist.

6. Resorbierbares Knochenersatzmaterial nach Anspruch 1 bis 5 **dadurch gekennzeichnet, dass**
die Kollagenmatrix zusätzlich ein SiO₂-Gel enthält.

7. Resorbierbares Knochenersatzmaterial nach Anspruch 4 bis 6 **dadurch gekennzeichnet, dass**
der interkonnektierend poröse Körper eine innere Stützstruktur besitzt.

8. Resorbierbares Knochenersatzmaterial nach Anspruch 7 **dadurch gekennzeichnet, dass**
die Stützstruktur aus einem Metall, vorzugsweise Titan besteht.

9. Resorbierbares Knochenersatzmaterial nach Anspruch 7 **dadurch gekennzeichnet, dass**
die Stützstruktur aus einem resorbierbaren Metall besteht.

10. Resorbierbares Knochenersatzmaterial nach Anspruch 7 **dadurch gekennzeichnet, dass**
die Stützstruktur aus einem resorbierbaren organischen Material besteht.

11. Resorbierbares Knochenersatzmaterial nach Anspruch 4 bis 6 **dadurch gekennzeichnet, dass**
der interkonnektierend poröse Körper derart ausbildet ist, dass dessen innere Oberfläche durch Kollagen bedeckt ist, das örtlich bestimmt, vernetzt ist.

## Claims

1. Resorbable bone replacement material consisting of calcium phosphate particles of different phases, and collagen, **characterized in that**
a) said collagen is crosslinked via bridge molecules selected from the group comprising a monocyclic dihydroxyaromatic compound, a bicyclic dihydroxyaromatic compound, a polycyclic dihydroxyaromatic compound, a bicyclic trihydroxyaromatic compound or a polycyclic trihydroxyaromatic compound, wherein the hydroxyaromatic compounds are not part of a polymer chain, such that a matrix is formed in which shorter chained peptides, particularly lysine containing peptides are additionally embedded; and
b) the formation of said collagen matrix with said calcium phosphate particles of different phases and concentrations embedded therein occurs under the influence of polyphenol oxidases.

2. Resorbable bone replacement material according to claim 1, **characterized in that**
said lysine containing peptides are repeating dipeptide units containing lysine.

3. Resorbable bone replacement material according to claim 1 or claim 2,
**characterized in that**
polymers exclusively consisting of two amino acids are incorporated as said shorter chained peptides, in a particularly advantageous manner (lysine-tyrosine)ₙ, wherein n takes values between 5 and 40.

4. Resorbable bone replacement material according to claims 1 to 3,
**characterized in that**
said bone replacement material forms an interconnectingly porous body.

5. Resorbable bone replacement material according to claim 4, **characterized in that**
said bone replacement material is embodied as a molded article.

6. Resorbable bone replacement material according to claims 1 to 5,
**characterized in that**
said collagen matrix additionally contains a SiO₂ gel.

7. Resorbable bone replacement material according to claims 4 to 6, **characterized in that**
said interconnectingly porous body has an internal support structure.

8. Resorbable bone replacement material according to claim 7, **characterized in that**
said support structure consists of a metal, preferably titanium.

9. Resorbable bone replacement material according to claim 7, **characterized in that**
said support structure consists of a resorbable metal.

10. Resorbable bone replacement material according to claim 7, **characterized in that**
said support structure consists of a resorbable organic material.

11. Resorbable bone replacement material according to claims 4 to 6,
**characterized in that**
said interconnectingly porous body is formed in such a way that its inner surface is covered by collagen which is crosslinked in a locally determined manner.

## Revendications

1. Matériau osseux de substitution résorbable constitué par des particules de phosphate de calcium de différentes phases et par du collagène, **caractérisé en ce que**
a) le collagène est réticulé par des molécules de pontage choisies parmi un groupe qui comprend un composé dihydroxy aromatique monocyclique, un composé dihydroxy aromatique bicyclique, un composé dihydroxy aromatique polycyclique, un composé trihydroxy aromatique bicyclique ou un composé trihydroxy aromatique polycyclique, les composés hydroxy aromatiques ne faisant pas partie d'une chaîne polymère, de telle sorte qu'il est réalisé une matrice dans laquelle sont additionnellement noyés des peptides à chaîne plus courte, en particulier des peptides contenant de la lysine, et
b) la réalisation de la matrice de collagène avec des particules de phosphate de calcium de différentes phases et concentration noyées dans celle-ci est effectuée sous l'influence de polyphénol oxydases.

2. Matériau osseux de substitution résorbable selon la revendication 1,
**caractérisé en ce que**
les peptides contenant de la lysine sont des unités dipeptidiques répétitives contenant de la lysine.

3. Matériau osseux de substitution résorbable selon la revendication 1 ou 2,
**caractérisé en ce que**
on incorpore, en tant que peptides à chaîne plus courte, exclusivement des polymères constitués par deux aminoacides, de manière particulièrement avantageuse (lysine-thyrosine)ₙ, n prenant des valeurs entre 5 et 40.

4. Matériau osseux de substitution résorbable selon les revendications 1 à 3,
**caractérisé en ce que**
le matériau osseux de substitution forme un corps poreux interconnecté.

5. Matériau osseux de substitution résorbable selon la revendication 4, **caractérisé en ce que**
celui-ci est réalisé sous forme de corps moulé.

6. Matériau osseux de substitution résorbable selon les revendications 1 à 5,
**caractérisé en ce que**
la matrice de collagène contient additionnellement un gel de SiO₂.

7. Matériau osseux de substitution résorbable selon les revendications 4 à 6,
**caractérisé en ce que**
le corps poreux interconnecté possède une structure de renfort intérieure.

8. Matériau osseux de substitution résorbable selon la revendication 7, **caractérisé en ce que**
la structure de renfort est en métal, de préférence en titane.

9. Matériau osseux de substitution résorbable selon la revendication 7, **caractérisé en ce que**
la structure de renfort est en un matériau résorbable.

10. Matériau osseux de substitution résorbable selon la revendication 7,
**caractérisé en ce que**
la structure de renfort est en un matériau organique résorbable.

11. Matériau osseux de substitution résorbable selon les revendications 4 à 6,
**caractérisé en ce que**
le corps poreux interconnecté est réalisé de telle sorte que sa surface intérieure est recouverte par du collagène qui est réticulé de manière localement déterminée.
